# EUROPEAN PATENT SPECIFICATION

(11) **EP 1 863 438 B1**
(45) Date of publication and mention of the grant of the patent: **24.10.2018**
(21) Application number: 06716433.5
(22) Date of filing: 17.03.2006
(51) Int. Cl.: A61K 8/37, A61K 8/06, A61K 8/34, A61K 8/81, A61Q 11/00, A61K 8/22, A61K 8/86, A61K 8/73

(54) **TOOTH WHITENER**
ZAHNAUFHELLER
SYSTEME DE BLANCHIMENT DES DENTS

(30) Priority: 18.03.2005 KR 20050022510; 12.05.2005 KR 20050039552
(43) Date of publication of application: 12.12.2007
(73) Proprietor: LG Household & Health Care Ltd., Seoul 150-010 (KR)
(72) Inventor: KWAK, Sang-Hoon, Daejeon 302-120 (KR); YUN, Sei-Young, Seoul 158-050 (KR); KIM, Han-Soo, Daejeon 306-050 (KR); CHANG, Sug-Youn, Seoul 150-993 (KR); KIM, Sang-Nyun, Daejeon 305-728 (KR); MOON, Kyo-Tae, Chungcheongnam-do 339-843 (KR)
(74) Representative: Schwahn, Hartmut
(86) International application number: PCT/KR2006/000983
(87) International publication number: WO 2006/098602

(56) References cited:
- EP-A1- 0 429 224
- GB-A- 2 139 919
- KR-A- 20050 046 050
- US-A1- 2003 152 528
- US-B1- 6 221 341
- US-B1- 6 296 834
- LEE J. ET AL.: 'Water Quantitatively Induces the Mucoadhesion of Liquid Crystalline Phases of Glyceryl Monooleate' JOURNAL OF PHARMACY AND PHARMACOLOGY vol. 53, 2001, pages 629 - 636, XP008121001

## Description

### Technical Field

The present invention relates to a tooth whitener, according to claim 1, and more particularly to a tooth whitener that is easy to spread in a liquid form when being applied to teeth, becomes viscous when moisture, *e.g.,* saliva, is introduced thereinto, thus enabling adherence and fixing to the teeth, and allows a whitening substance to be released to the surface of the teeth while being attached to the teeth, thereby exerting its teeth whitening effects.

### Background Art

In general, a tooth consists of an inner dentin layer and an outer enamel layer. The reasons why teeth turn yellow are that substances having chromophores, *e.g.,* double bonds, originating from foreign materials, *e.g.,* food, are deposited on the outer enamel layer of teeth ("internal factors"), and that scales and soft deposits are attached to the surface of teeth to cause discoloration of the teeth ("external factors"). The tooth discoloration caused due to the external factors can be readily solved by brushing, but the tooth discoloration caused due to the internal factors cannot be solved by the use of toothpaste and by brushing. Accordingly, there is a limitation in achieving teeth whitening effects using toothpaste.

Under such circumstances, tooth whitening techniques using a hydrogen peroxide solution have been developed. In 1960, an American dentist unexpectedly discovered during the treatment of the gums of his patients that a hydrogen peroxide solution whitens the teeth, and as a result, developed a tooth whitener. In the 1980's, teeth whitening devices for domestic use were developed. These teeth whitening devices are products in which a hydrogen peroxide or carbamide peroxide gel is contained in a dental tray. However, the devices have many problems with regard to safety within the oral cavity because the peroxide must be used at a high concentration and the tray causes physical irritation to the gums.

Thereafter, a number of efforts have been made to overcome the disadvantages of the conventional teeth whitening devices. For example, dental strip products containing peroxide at a low concentration and liquid products capable of being directly applied to teeth were developed. The liquid products have advantages in that they are convenient to use and readily spreadable on particular sites of teeth where users want to use them, but have drawbacks in that they tend to stick to teeth and soft tissues of the oral cavity, *e.g.,* lips, causing an irritation, and they are easily diluted by saliva, which makes absorption of hydrogen peroxide into the teeth difficult, resulting in poor teeth whitening effects.

In attempts to overcome the above problems, novel formulations have recently been introduced or filed as patent applications. For instance, Korean Patent Application Nos. 1999-7011451 and 2001-7011679 suggest systems in which a peroxide-containing gel is applied to a polyethylene strip. These systems offer advantages in that the peroxide can come into contact with users' teeth for a long period of time and the users can enjoy their casual social activities while wearing the tooth whitening strip. Although the systems provide an improved feeling of use when the peroxide-containing gel layer is attached to teeth, efficient delivery of the whitening ingredient between the teeth is not achieved, thus causing poor whitening efficacy. In addition, the systems can be attached to teeth, but there is a danger that the strip may be readily separated from the teeth due to the movement of a tongue or lips when saliva is introduced thereinto. Furthermore, when it is intended to attain selective whitening effects only at desired particular teeth, the efficiency is poor and the procedure is troublesome because the strip is fixed and is not deformed.

U.S. Patent No. 6,770,266, issued to Colgate, discloses a liquid tooth whitener using a polyethylene oxide. The tooth whitener offers convenience in use and little unnatural feeling, but has problems in that the use of the watersoluble polymer (*i.e.* polyethylene oxide) causes poor adhesion to teeth and easy dilution of the tooth whitener in saliva. As a result, the absorption of peroxide in the teeth is insufficient and thus whitening effects required by consumers are not achieved.

U.S. Patent No. 6,569,408, issued to P&G, discloses a liquid tooth whitener using an organosiloxane resin. Since this tooth whitener is convenient to use and has good adhesion to teeth, it can be used overnight. However, the tooth whitener has a disadvantage in that the use of the non-hydrophilic polymer impedes the absorption of peroxide as a whitening ingredient in teeth.

U.S. Patent No. 6,555,020, issued to Den-mat Corp., discloses a liquid tooth whitener using polyacrylic acid. However, the tooth whitening gel is readily diluted by saliva, causing poor adhesion to teeth. In addition, whitening reactions on the surface of teeth are retarded by the addition of a peroxide stabilizer, such as EDTA or CDTA, which makes it difficult to attain desired teeth whitening effects.

U.S. Patent Publication No. 2003/0152528, by Singh et al., discloses a tooth whitener using a hydrophilic polymer and an oligomer, having a molecular weight below about 1000 Da, capable of hydrogen bonding to the hydrophilic polymer. This tooth whitener is suitable for solid-phase formulations produced by casting and extrusion, but, in the case of liquid-phase formulations, has a disadvantage in that the use of low-molecular weight monomers only impedes the formation of strong hydrogen bonding to the hydrophilic polymer due to high intermolecular kinetic energy and increased degree of freedom on gels. Accordingly, the tooth whitener cannot be selectively attached to teeth.

Patent document GB 2139919 discloses stable high internal phase ratio emulsions as delivery systems for topical products.

On the other hand, glycerol monooleate, a fatty acid originating from vegetable oils, forms liquid crystals in response to temperature and moisture content, and it is known that its shape and physical properties may be varied depending on the ambient conditions. The crystal form of glycerol monooleate is changed according to changes in the moisture content of the glycerol monooleate. The crystal form of glycerol monooleate is changed from pure glycerol monooleate to a W/O emulsion (reversed micelles), a lamellar phase liquid crystal and a cubic phase liquid crystal with increasing moisture content. When the moisture content of glycerol monooleate further increases, cubic phase liquid crystals are separated to cause dissolution of a drug. At this time, the viscosity and adhesive force of formulations vary according to changes in the crystal structure of glycerol monooleate. That is, since glycerol monooleate in a lamellar phase, a W/O emulsion (reversed micelles) phase or a reversed hexagonal phase is flowable, it can be easily applied to the surface of teeth. As glycerol monooleate is changed to a cubic phase in structure with increasing temperature and moisture content, it is solidified and loses its flowability. These changes are known to be due to 1,2-diol groups positioned at a glycerol moiety of glycerol monooleate, which affect the formation of a liquid crystal structure. The same phenomenon also takes place in surfactants having a structure similar to that of glycerol monooleate.

However, double bonds present at an oleate moiety of glycerol monooleate are easily attacked by oxidants, such as hydrogen peroxide, and are then decomposed. Accordingly, it is known that glycerol monooleate is not compatible with peroxide (Handbook of Pharmaceutical Excipients, 4th edition, p 262).

### Disclosure

### Technical Problem

Therefore, the present invention has been made in view of the problems of the conventional tooth whiteners, and it is an object of the present invention to provide a tooth whitener having suitable physical properties that can achieve teeth whitening effects when being applied to teeth. That is, the tooth whitener of the present invention overcomes the disadvantages of conventional strip and liquid products and solves various problems resulting from the formulation of glycerol monooleate. The tooth whitener of the present invention is easily applied to desired sites within the oral cavity to achieve teeth whitening effects and improves the phase stability of glycerol monooleate and the stability of peroxide.

### Technical Solution

In accordance with an aspect of the present invention for achieving the above objects, there is provided a tooth whitener in W/O emulsion phase which comprises a glycerol monooleate, a polyol, a polymer, a peroxide and a hydrophilic solvent.

### Best Mode

The present invention provides a new type of tooth whitening system that is easily spreadable when being applied to teeth and is readily fixed to the teeth after being applied to the teeth so as to deliver an effective tooth whitening ingredient to the surface of the teeth. The tooth whitening system of the present invention is a formulation in which glycerol monooleate, which has been recognized as being difficult to use in conventional tooth whiteners, is formulated into a W/O emulsion phase to improve the phase stability of the glycerol monooleate and the stability of a peroxide.

The tooth whitener of the present invention comprises glycerol monooleate as a base. Glycerol monooleate forms liquid crystals in response to temperature and moisture content, and it is known that its shape and physical properties are varied depending on the ambient conditions. Since surfactants and glycerol monooleate in a lamellar phase, a W/O emulsion (reversed micelles) phase or a reversed hexagonal phase are flowable, they can be easily applied to the surface of teeth. However, as surfactants and glycerol monooleate are changed to a cubic phase in structure with increasing temperature and moisture content, they are solidified and lose their flowability.

The tooth whitener of the present invention utilizes such changes in the physical properties of liquid crystals. While the tooth whitener of the present invention is attached to teeth, an effective tooth whitening ingredient is slowly released to the surface of the teeth. At this time, saliva is absorbed into the tooth whitener, and thus the tooth whitener gradually becomes viscous. This increase in viscosity enables the control of contact time of the whitening ingredient with the teeth.

The tooth whitener of the present invention is a liquid formulation before being applied to teeth and is then adhered to the teeth by solidification after being applied to the teeth. In addition, the crystal form of the glycerol monooleate is changed from a W/O emulsion phase to a cubic phase after being applied to the teeth. As the content of saliva within the oral cavity increases, the cubic phase is separated to cause dissolution of hydrogen peroxide. Therefore, the solidified formulation is lost by the action of saliva present within the oral cavity without using a special removal process.

That is, the glycerol monooleate used as a base in the tooth whitener of the present invention serves to induce a phase transformation of the tooth whitener into a cubic phase under the internal conditions of the oral cavity. Specifically, the base (*i.e.* glycerol monooleate) is solidified by the inner temperature of the oral cavity and moisture, *e.g*., saliva, present in the oral cavity and the tooth whitener applied to teeth becomes viscous so that the tooth whitener is attached and fixed to the teeth. Accordingly, the tooth whitener of the present invention is suitably flowable and is convenient to handle before being applied to teeth. In addition, the tooth whitener of the present invention becomes rapidly viscous by the introduction of saliva thereinto after being applied to the teeth so that it is well fixed and attached to the teeth. In addition, the tooth whitener of the present invention is not readily diluted or lost under the conditions within the oral cavity.

In the tooth whitener of a W/O emulsion phase according to the present invention, the glycerol monooleate can be added in an amount of about 15 to about 95% by weight. For shape maintenance of a formulation, the glycerol monooleate is preferably added in an amount of 30-85% by weight. When the glycerol monooleate is present in an amount of less than 15% by weight relative to the total weight of the composition, the glycerol monooleate is present in a cubic phase within the formulation, making it difficult to spread on teeth and damaging the stability of hydrogen peroxide. Meanwhile, when the glycerol monooleate is present in an amount exceeding 95% by weight relative to the total weight of the composition, the viscosity of the formulation is excessively varied according to changes in temperature, adversely affecting the phase stability of the formulation.

The glyceryl moiety of the glycerol monooleate is hydrophilic, and the monooleate moiety of the glycerol monooleate is lipophilic. Accordingly, it is necessary to prepare the tooth whitener of the present invention in a W/O emulsion type so that a hydrophilic material, such as hydrogen peroxide, is selectively present in the glyceryl moiety. As a whitening ingredient used in the tooth whitening, a 35% hydrogen peroxide solution or an aqueous peroxide solution is commonly used. A 2% or higher peroxide solution is preferably used to attain satisfactory teeth whitening effects. In general, a 6% or higher hydrophilic ingredient is required to prepare a 2% hydrogen peroxide solution from a 35% hydrogen peroxide solution, which is most preferably used for tooth whitening. In this case, lamellar phase liquid crystals are formed instead of a W/O emulsion phase, resulting in poor stability of the tooth whitener.

Polyol can be used to solve poor stability of the tooth whitener. The use of polyol permits the tooth whitener to have a W/O emulsion phase. As mentioned earlier, 1,2-diol groups of the glyceryl moiety of the glycerol monooleate form liquid crystals due to the presence of moisture or intramolecular hydrogen bonding. To inhibit the formation of crystals, there can be used a low-molecular weight polymer, preferably polyol, having hydroxyl (-OH) groups capable of competing with 1,2-diol groups of the glyceryl moiety of the glycerol monooleate. Since hydroxyl groups present in polyol compete with 1,2-diol groups of the glycerol monooleate, the formation of liquid crystals having a matrix phase can be prevented despite an increase in moisture content. Generally, when a 5% or higher hydrophilic material is contained in a matrix of the glycerol monooleate to which no hydroxyl group is added, liquid crystals are observed. In contrast, when polyol with hydroxyl groups is added, W/O emulsion can be maintained at a level of about 15%.

Polyol having hydroxyl groups competitively impedes the intramolecular bonding of 1,2-diol groups of the glycerol monooleate and bonding of the 1,2-diol groups with water, thus preventing the formation of liquid crystals and enabling the formation of a W/O emulsion (reversed micelles). When the tooth whitener of the present invention is prepared in a W/O emulsion phase (reversed micelles), the phase stability of the glycerol monooleate and the stability of a peroxide as a main ingredient over time can be improved.

The use of polyol enables control of gelling time. That is, a low-molecular weight polyol having hydroxyl (-OH) groups capable of competing with 1,2-diol groups of the glycerol monooleate is added to retard the formation rate of crystals.

In addition, since the addition of polyol drops the freezing point and increases the hardening point of the glycerol monooleate, changes in the viscosity of the glycerol monooleate with varying temperature, which may be the largest obstacle to the commercialization of glycerol monooleate, can be advantageously prevented.

That is, the use of a polyol is important in the control over the physical properties of the W/O emulsion phase tooth whitener according to the present invention, particularly, rate of viscosity changes of the tooth whitener before and after being applied to teeth. The rate of viscosity change of the tooth whitener may be controlled by varying the content of polyol.

The polyol used in the present invention preferably has a molecular weight not higher than 8,000. In addition to the above functions, the polyol functions to hinder the intermolecular arrangement of crystals with decreasing temperature, thus preventing changes in phase with varying temperature during production and circulation.

Furthermore, the polyol forms intramolecular bonds with 1,2-diol groups of the glycerol monooleate playing an important role in forming liquid crystals, or impedes bonding with water. The polyol bonds with the glycerol monooleate to loosen bonds between liquid crystals of the glycerol monooleate, thus increasing the release rate of a drug.

Examples of suitable polyols that can be used in the tooth whitener of the present invention include polyethylene glycol, polypropylene glycol, propylene glycol, glycerin, stearyl alcohol, xylitol, sorbitol, mannitol, and mixtures thereof. Preferred are polyethylene glycol and polypropylene glycol.

The amount of the polyol used in the tooth whitener of the present invention is in the range of 0.05-20% by weight and preferably 1-10% by weight, based on the total weight of the composition. When the polyol is used in an amount of less 0.05% by weight, the viscosity control and the preventive effects against phase change in response to temperature cannot be expected. Meanwhile, when the polyol is used in an amount exceeding 20% by weight, the hydroxyl groups of the polyol hinders the formation of liquid crystals of the glycerol monooleate, and as a result, phase changes from a W/O emulsion phase to a cubic phase, which are characteristics required in the tooth whitener of the present invention, are not attained.

A hydrophilic solvent, *e.g.,* water or ethanol, plays a key important role in determining various phases of the glycerol monooleate. The control over the moisture content is required to maintain a W/O emulsion phase suitably employed in the present invention, leading to easy formulation of the tooth whitener. The content of the hydrophilic solvent in the W/O emulsion phase tooth whitener of the present invention is in the range of 1-50% by weight and preferably 2-20% by weight, based on the total weight of the composition. When the content of the hydrophilic solvent is less than 1% by weight, the viscosity of the tooth whitener is too high and thus it is not easy to spread the tooth whitener on teeth. Meanwhile, when the content of the hydrophilic solvent is more than 50% by weight, the phase of the glycerol monooleate in the tooth whitener is cubic, which causes poor adhesion of the tooth whitener to teeth and deteriorates the stability of hydrogen peroxide.

Examples of suitable tooth whitening ingredients that can be used in the tooth whitener of the present invention include, but are not limited to: hydrogen peroxide; peroxide salts, such as percarbonates and perborates; urea peroxide; tetrasodium pyrophosphate perphosphate; polymers, such as polyvinylpyrrolidone-hydrogen peroxide complexes; and inorganic metal peroxide salts, such as calcium peroxide (CaO₂), barium peroxide (BaO₂), magnesium peroxide (MgO₂), sodium peroxide (Na₂O₂) and potassium peroxide (Ka₂O₂). For better teeth whitening effects, these whitening ingredients can be used alone or in combination thereof. The tooth whitening ingredient can be added in an amount of 0.5-50% by weight and preferably 1-30% by weight, based on the total weight of the composition according to the present invention. If the peroxide is a solid or powder, the peroxide is previously dissolved or dispersed in water to prepare a more homogeneous tooth whitener.

The tooth whitener of the present invention comprises a polymeric compound, *i.e.* a polymer, to control the physical properties of the base (*i.e.* glycerol monooleate). For example, a polymer having superior adhesion to teeth can be added to increase the adhesion of the tooth whitener to teeth. The polymer can be used in an amount of 0.2-40% by weight, based on the total weight of the tooth whitener according to the present invention. For maintenance of the shape of the tooth whitener, it is preferred to use the polymer in an amount of 0.2-10% by weight. When the polymer is used in an amount of less than 0.2% by weight relative to the total weight of the composition, the adhesion of the tooth whitener to teeth is poor and thus the desired effects cannot be exhibited. Meanwhile, when the polymer is used in an amount exceeding 40% by weight, the polymer is precipitated and thus the phase stability is worsened.

Examples of suitable polymers that can be used in the W/O emulsion phase tooth whitener of the present invention include the following polymers: non-ionic polymers, such as polyvinyl alcohol, Poloxamer, polyvinyl pyrrolidone, polyvinyl pyrrolidone/vinyl acetate copolymers, hydroxypropyl cellulose, hydroxypropylmethyl cellulose, hydroxyethyl cellulose, hydroxypropylethyl cellulose, and Polyox; anionic polymers, such as carboxymethyl cellulose, polymers consisting of carboxypropyl cellulose and its salts, xanthan gum, carrageenan gum, and alginate gum; other polymers, such as karayan gum, arabic gum and its salt derivatives, and gelatin; synthetic polymers, such as polyacrylic acid, Carbopol, polyquaternium-11, polyquaternium-39, polyalkylvinylether-maleic acid (PVM/MA) copolymers (Gantrez AN 119, AN 139, S-97); and mixtures thereof.

The tooth whitener of the present invention may further comprise a stabilizer having a function to adjust the pH of oral care products, particularly, tooth whiteners, while advantageously stabilizing the peroxide. The stabilizer serves to inhibit variation in pH during storage of the tooth whitener and to maintain the pH value so as not to irritate the oral cavity. As the stabilizer, there can be used a metal-complexing agent having superior compatibility with the peroxide. Examples of suitable stabilizers include polyphosphates, such as tetrasodium pyrophosphate (TSPP), sodium acid pyrophosphate (SAPP), sodium hexametaphosphate (SHMP), sodium tripolyphosphate (STP), sodium potassium tripolyphosphate (SKTP), tetrapotassium pyrophosphate (TKPP), acidic sodium meta-polyphosphate, and acidic sodium polyphosphate; and metal-chelating agents, such as ethylenediaminetetraacetate, aminotrimethylenephosphate and its salts, hydroxyethylenediphosphonate and its salts, ethylenediaminetetramethylenephosphonate and its salts, and diethylenetriaminepentamethylenephosphonate and its salts. These stabilizers may be used alone or in combination thereof.

The stabilizer having a pH-adjustment function is preferably used in an amount of 0.1-10% by weight relative to the weight of the peroxide. Particularly, since polyphosphates are known to be effective in the prevention of scale formation and removal of scales, they are thought to contribute to an improvement in whitening effects. In actuality, it could be confirmed that polyphosphate-containing tooth whiteners were effective in cleaning the surface and sides of teeth.

In addition to the aforementioned ingredients, the tooth whitener of the present invention may further comprise at least one additive selected from flavors, bleaching activators and dental caries inhibitors, and therapeutics against periodontal diseases. These additives serve to improve feeling of use without affecting the shape of the formulation and the stability of the peroxide. Representative flavors include peppermint, spearmint, menthol, citrus, herbs, and the like. Representative bleaching activators include titanium oxide, transition metal-complexes, tetraacetyl ethylene diamine (TAED), and the like. Representative dental caries inhibitors include sodium fluoride (NaF), calcium fluoride (CaF₂), and the like. Representative therapeutics against periodontal diseases include bamboo-salt, tocopherol acetate, vitamin D, alantoin chlorohydroxy aluminum, triclosan, xylitol, calcium glycerol phosphate, aminocaproic acid, tronic amine, KNO₃, monofluorophosphate, enzymes, and the like.

The new type of delivery system for tooth whitening comprising the above-mentioned ingredients offers improved feeling of use and additional functions.

Advantages of the tooth whitener according to the present invention are summarized below:
Firstly, since the tooth whitener of the present invention is present in a W/O emulsion (reversed micelles) or lamellar liquid crystal phase before use, it is convenient to spread. The tooth whitener of the present invention reacts with moisture when being attached to teeth to form a cubic phase liquid crystal structure, which allows tooth whitening ingredients to be efficiently delivered between the teeth.

Secondly, the addition of polyol having hydroxyl groups to glycerol monooleate increases the capability to form a W/O emulsion (reversed micelles). This advantage enables provision of a tooth whitener using glycerol monooleate and a peroxide, which have been recognized as being impossible in conventional tooth whiteners.

Thirdly, the addition of polyol having hydroxyl groups to glycerol monooleate minimizes changes in viscosity of the tooth whitener with varying temperature and enables control over the release rate of a drug.

### Mode for Invention

The present invention will now be described in more detail with reference to the following preferred examples. However, these examples are given for the purpose of illustration and are not intended to limit the present invention. In the examples, percentages (%) are by weight.

### Example 1

| | |
|---|---|
| Hydrogen peroxide | 3% |
| Glycerol monooleate | 77% |
| Poloxamer | 1% |
| Tetrasodium pyrophosphate | 0.1% |
| Flavor | 0.9% |
| Propylene glycol | 1% |
| Polyethylene glycol 400 | 1% |
| Polyvinyl pyrrolidone | 1% |
| Distilled water | 15% |

While the glycerol monooleate was liquefied at around 50°C, the other ingredients were added thereto. At this time, the hydrogen peroxide and the distilled water were finally added. The resulting mixture was homogenized to prepare a W/O emulsion phase tooth whitener of the present invention.

### Example 2

| | |
|---|---|
| Hydrogen peroxide | 3% |
| Glycerol monooleate | 77% |
| Tetrasodium pyrophosphate | 0.1% |
| Flavor | 0.9% |
| Polyvinyl pyrrolidone | 6% |
| Distilled water | 11.5% |
| Polyethylene glycol | 1.5% |

While the glycerol monooleate was liquefied at around 50°C, the other ingredients were added thereto. At this time, the hydrogen peroxide and the distilled water were finally added. The resulting mixture was homogenized to prepare a liquid crystal type tooth whitener of the present invention.

### Example 3

| | |
|---|---|
| Sodium percarbonate | 10% |
| Glycerol monooleate | 67% |
| Polyvinyl pyrrolidone | 3% |
| Polyethylene glycol 8000 | 5% |
| Sodium fluoride | 2% |
| Distilled water | 4% |
| Flavor | 5% |
| Polypropylene glycol | 1% |
| Sodium acid pyrophosphate | 3% |

While the glycerol monooleate was liquefied at around 50°C, the other ingredients except the sodium acid pyrophosphate were added thereto. At this time, the distilled water was finally added. The resulting mixture was homogenized to prepare a W/O emulsion phase tooth whitener of the present invention.

### Example 4

| | |
|---|---|
| Sodium percarbonate | 10% |
| Glycerol monooleate | 67% |
| Carbomer | 8% |
| Polyethylene glycol 8000 | 1% |
| Distilled water | 10% |
| Flavor | 1% |
| Sodium acid pyrophosphate | 3% |

While the glycerol monooleate was liquefied at around 50°C, the other ingredients except the sodium acid pyrophosphate were added thereto. At this time, the distilled water was finally added. The resulting mixture was homogenized to prepare a liquid crystal type tooth whitener of the present invention.

### Example 5

| | |
|---|---|
| Urea peroxide | 15% |
| Glycerol monooleate | 70% |
| Hydroxypropyl cellulose | 4% |
| Tetrasodium pyrophosphate | 2% |
| Flavor | 0.5% |
| Titanium oxide | 0.1% |
| Distilled water | 3% |
| Ethanol | 0.4% |
| Propylene glycol | 2% |
| Polyethylene glycol | 3% |

While the glycerol monooleate was liquefied at around 50°C, the other ingredients were added thereto. At this time, urea peroxide and distilled water was finally added. The resulting mixture was homogenized to prepare a W/O emulsion phase tooth whitener of the present invention.

### Example 6

| | |
|---|---|
| Urea peroxide | 15% |
| Glycerol monooleate | 70% |
| Hydroxypropyl cellulose | 4% |
| Tetrasodium pyrophosphate | 2% |
| Flavor | 0.5% |
| Titanium oxide | 0.1% |
| Distilled water | 3% |
| Ethanol | 0.4% |
| Propylene glycol | 5% |

While the glycerol monooleate was liquefied at around 50°C, the other ingredients were added thereto. At this time, the urea peroxide and the distilled water were finally added. The resulting mixture was homogenized to prepare a liquid crystal type tooth whitener of the present invention.

### Comparative Example 1

A tooth whitener was prepared in the same manner as in Example 1, except that olive oil was used instead of polyethylene glycol and propylene glycol.

### Comparative Example 2

A tooth whitener was prepared in the same manner as in Example 2, except that no polyvinyl pyrrolidone was used and instead distilled water was used.

### Comparative Example 3

A semi-solid tooth whitener was prepared using the following composition:

| | |
|---|---|
| Organosiloxane resin | 25% |
| Silicone gum Mixture of ethyl acetate | 4.2% |
| and propyl acetate | 17% |
| Bentone clay | 1% |
| Sodium percarbonate | 17% |

### Comparative Example 4

A tooth whitener was prepared in the same manner as in Example 2, except that no polyethylene glycol was used and instead distilled water was used.

### Experimental Example 1: Measurement of changes in viscosity

Changes in the viscosity of the formulation prepared in Example 1 before and after moisture was introduced into the formulation were measured by RVT using a Brookfield viscometer. The results are shown in Table 1 below.

**TABLE 1**

| | Viscosity (cps) before introduction of moisture | Viscosity (cps) after introduction of moisture |
|---|---|---|
| Example 1 | 50000∼70000 | 400000∼500000 |
| Comparative Example 1 | 100000∼150000 | 400000∼500000 |

The results of Table 1 show that the addition of the polyol reduces the viscosity of the formulation according to the present invention so that the formulation is easily spreadable and the introduction of moisture increases the viscosity of the formulation, indicating that the characteristics of the hardened glycerol monooleate are not lost.

### Experimental Example 2: Dissolution test

A dissolution test was conducted using a phosphate buffer solution in accordance with the dissolution test method described in the U.S. pharmacopoeia. Specifically, the dissolution test was conducted on each of the formulations prepared in Example 1 and Comparative Examples 1, 3 and 4 using 500 ml of a phosphate buffer solution at 35°C while rotating a paddle at 25 rpm. The content of hydrogen peroxide in the buffer solution was measured and the dissolution rate of the hydrogen peroxide from the formulation was calculated. The results are shown in Table 2 below.

**TABLE 2**

| Time | 5 minutes | 30 minutes | 1 hour |
|---|---|---|---|
| Example 1 | 32 ± 5% | 53 ± 15% | 85 ± 13% |
| Comparative Example 1 | 20 ± 4% | 45 ± 7% | 67 ± 10% |
| Comparative Example 3 | 15 ± 5% | 25 ± 7% | 40 ± 10% |
| Comparative Example 4 | 16 ± 5% | 33 ± 7% | 55 ± 10% |

From the results of Table 2, it can be confirmed that the formulation prepared in Example 1 has a higher release rate than the formulations prepared in Comparative Examples, which indicates that the release rate of the drug can be controlled by varying the content of the polyol in the formulation of the present invention.

### Experimental Example 3: Changes in viscosity with varying temperature

Changes in the viscosity of the formulation prepared in Example 1 according to changes in temperature were measured by RVT using a Brookfield viscometer. The results are shown in Table 3 below.

**TABLE 3**

| Temp. | 5°C | 30°C | 45°C |
|---|---|---|---|
| Example 1 | 50000∼70000 | 50000∼70000 | 50000∼70000 |
| Comparative Example 1 | 700000∼850000 (Hardened) | 100000∼150000 | 1000∼10000 |

As can be seen from the data shown in Table 3, the addition of the polyol minimizes changes in the viscosity of the glycerol monooleate with increasing temperature.

### Experimental Example 4: Stability test of peroxide

The formulations prepared in Example 2 and Comparative Examples 2 and 3 were titrated in accordance with the peroxide quantification method described in the Korean pharmacopoeia. Thereafter, the stability of each of the formulations was evaluated during storage at room temperature for 1, 2 and 6 months. The data shown in Table 4 represent the peroxide contents at the respective time points.

**TABLE 4**

| Time | 1 month | 2 month | 6 month |
|---|---|---|---|
| Example 2 | 97 ± 2% | 96 ± 1% | 94 ± 3% |
| Comparative Example 2 | 91 ± 4% | 85 ± 2% | 67 ± 5% |
| Comparative Example 3 | 95 ± 4% | 91 ± 5% | 88 ± 3% |

The results of Table 4 confirm that the formulation prepared in Example 2 has superior stability over time when compared to the formulations prepared in Comparative Examples.

### Experimental Example 5: Test for whitening efficacy

The formulations prepared in Examples 2 and 4 and Comparative Example 3 were tested *in vitro* for whitening effects. For the test, discolored hydroxyapatite (HAP) tablet specimens were used. The formulations were applied to the discolored specimens, allowed to stand at room temperature for one minute, and dipped in water for one minute. After the specimens were taken out of the water, they were wiped three times with a tissue. Thereafter, the dry specimens were allowed to stand in a thermo-hygrostat at a temperature of 37°C and a humidity of 95% for 6 hours, and dried at room temperature. L values of the specimens were measured. Difference in the L values, ΔL, before and after the application was calculated for each specimen, and the results are shown in Table 5 below.

**TABLE 5**

| Example No. | ΔL (Once) |
|---|---|
| Example 2 | 37.50 ± 4.50 |
| Example 4 | 37.96 ± 1.98 |
| Comparative Example 3 | 15.08 ± 0.55 |

It appears from the results of Table 5 that the liquid crystal type tooth whiteners prepared using glycerol monooleate in Examples 2 and 4 show superior whitening effects when compared to the formulation prepared using an organosiloxane resin in Comparative Example 3.

### Industrial Applicability

As apparent from the above description, the tooth whitener of the present invention comprises glycerol monooleate as a base. The tooth whitener of the present invention is flowable before being applied to teeth and is spreadable when being applied to teeth. In addition, the tooth whitener of the present invention is solidified by the action of moisture, such as saliva, after being applied to teeth, and can thus be adhered and fixed to the teeth. Furthermore, changes in viscosity of the tooth whitener with varying temperature can be minimized and the release rate of a whitening ingredient can be controlled by the addition of polyol.

## Claims

1. A tooth whitener in W/O emulsion phase which comprises a glycerol monooleate, a polyol, a polymer, a peroxide and a hydrophilic solvent, wherein the glycerol monooleate is in an amount of 15 to 95% by weight, of the composition the polyol is in an amount of 0.05 to 20% by weight, of the composition and the polyol is polyethylene glycol, polypropylene glycol, propylene glycol, glycerin, stearyl alcohol, xylitol, sorbitol, mannitol, or a mixture thereof.

2. The tooth whitener according to claim 1, wherein the polyol has a molecular weight of 8,000 or less.

3. The tooth whitener according to claim 1, wherein the polymer is polyvinyl alcohol, Poloxamer, polyvinyl pyrrolidone, a polyvinyl pyrrolidone/vinyl acetate copolymer, hydroxypropyl cellulose, hydroxypropylmethyl cellulose, hydroxyethyl cellulose, hydroxypropylethyl cellulose, Polyox, carboxymethyl cellulose, a polymer consisting of carboxypropyl cellulose or its salt, xanthan gum, carrageenan gum, alginate gum, karayan gum, arabic gum or its salt derivative, gelatin, polyacrylic acid, Carbopol, polyquaternium-11, polyquaternium-39, a polyalkylvinylether-maleic acid (PVM/MA) copolymer, or a mixture thereof.

4. The tooth whitener according to claim 1, wherein the peroxide is hydrogen peroxide, percarbonate, perborate, urea peroxide, tetrasodium pyrophosphate perphosphate, a polyvinylpyrrolidone-hydrogen peroxide complex, calcium peroxide (CaO₂), barium peroxide (BaO₂), magnesium peroxide (MgO₂), sodium peroxide (Na₂O₂), potassium peroxide (Ka₂O₂), or a mixture thereof.

5. The tooth whitener according to claim 1, wherein the glycerol monooleate is in an amount of 30 to 85% by weight. of the composition

6. The tooth whitener according to claim 1, wherein the polyol is in an amount of 1 to 10% by weight, of the composition

7. The tooth whitener according to claim 1, wherein the polymer is in an amount of 0.2 to 40% by weight. of the composition

8. The tooth whitener according to claim 7, wherein the polymer is in an amount of 0.2 to 10% by weight. of the composition

9. The tooth whitener according to claim 1, wherein the hydrophilic solvent is water or ethanol, and is in an amount of 1 to 50% by weight.

10. The tooth whitener according to claim 9, wherein the hydrophilic solvent is in an amount of 2 to 20% by weight. of the composition

11. The tooth whitener according to claim 1 which further comprises a peroxide stabilizer.

12. The tooth whitener according to claim 11, wherein the peroxide stabilizer has a pH-adjustment function, and is tetrasodium pyrophosphate (TSPP), sodium acid pyrophosphate (SAPP), sodium hexametaphosphate (SHMP), sodium tripolyphosphate (STP), sodium potassium tripolyphosphate (SKTP), tetrapotassium pyrophosphate (TKPP), acidic sodium meta-polyphosphate, acidic sodium polyphosphate, ethylenediaminetetraacetate, aminotrimethylenephosphate or its salt, hydroxyethylenediphosphonate or its salt, ethylenediaminetetramethylenephosphonate or its salt, diethylenetriaminepentamethylenephosphonate or its salt, or a mixture thereof.

13. The tooth whitener according to claim 12, wherein the peroxide stabilizer is in an amount of 0.1 to 10% by weight based on the weight of peroxide.

## Patentansprüche

1. Zahnbleichmittel in einer Wasser-in-Öl-Emulsion, enthaltend ein Glycerolmonooleat, ein Polyol, ein Polymer, ein Peroxid und ein hydrophiles Lösungsmittel, wobei das Glycerolmonooleat in einem Anteil von 15 bis 95 Gew.-% der Zusammensetzung vorliegt, das Polyol in einem Anteil von 0,05 bis 20 Gew.-% der Zusammensetzung vorliegt und das Polyol Polyethylenglykol, Polypropylenglykol, Propylenglykol, Glycerin, Stearylalkohol, Xylitol, Sorbitol, Mannitol oder eine Mischung davon ist.

2. Zahnbleichmittel nach Anspruch 1, wobei das Polyol ein Molekulargewicht von 8000 oder weniger besitzt.

3. Zahnbleichmittel nach Anspruch 1, wobei das Polymer Polyvinylalkohol, Poloxamer, Polyvinylpyrrolidon, ein Copolymer aus Polyvinylpyrrolidon und Vinylacetat, Hydroxypropylcellulose, Hydroxypropylmethylcellulose, Hydroxyethylcellulose, Hydroxypropylethylcellulose, Polyox, Carboxymethylcellulose, ein Polymer bestehend aus Carboxypropylcellulose oder dessen Salz, Xanthan, Carrageenan, Alginat, Karayan, Gummi arabicum oder dessen Salzderivat, Gelatine, Polyacrylsäure, Carbopol, Polyquaternium-11, Polyquaternium-39, ein Copolymer aus Polyalkylvinylether und Maleinsäure (PVM/MA) oder eine Mischung davon ist.

4. Zahnbleichmittel nach Anspruch 1, wobei das Peroxid Wasserstoffperoxid, Percarbonat, Perborat, Harnstoffperoxid, Tetranatriumpyrophosphatperphosphat, ein Komplex aus Polyvinalpyrrolidon und Wasserstoffperoxid, Calciumperoxid (CaO₂), Bariumperoxid (BaO₂), Magnesiumperoxid (MgO₂), Natriumperoxid (Na₂O₂), Kaliumperoxid (Ka₂O₂) oder eine Mischung davon ist.

5. Zahnbleichmittel nach Anspruch 1, wobei das Glycerolmonooleat in einem Anteil von 30 bis 85 Gew.-% der Zusammensetzung vorliegt.

6. Zahnbleichmittel nach Anspruch 1, wobei das Polyol in einem Anteil von 1 bis 10 Gew.-% der Zusammensetzung vorliegt.

7. Zahnbleichmittel nach Anspruch 1, wobei das Polymer in einem Anteil von 0,2 bis 40 Gew.-% der Zusammensetzung vorliegt.

8. Zahnbleichmittel nach Anspruch 7, wobei das Polymer in einem Anteil von 0,2 bis 10 Gew.-% der Zusammensetzung vorliegt.

9. Zahnbleichmittel nach Anspruch 1, wobei das hydrophile Lösungsmittel Wasser oder Ethanol ist und in einem Anteil von 1 bis 50 Gew.-% vorliegt.

10. Zahnbleichmittel nach Anspruch 9, wobei das hydrophile Lösungsmittel in einem Anteil von 2 bis 20 Gew.-% der Zusammensetzung vorliegt.

11. Zahnbleichmittel nach Anspruch 1, weiter enthaltend ein Peroxidstabilisierungsmittel.

12. Zahnbleichmittel nach Anspruch 11, wobei das Peroxidstabilisierungsmittel eine pH-Einstellfunktion besitzt und Tetranatriumpyrophosphat (TSPP), Dinatriumdihydrogenpyrophosphat (SAPP), Natriumhexametaphosphat (SHMP), Pentanatriumtriphosphat (STP), Natriumkaliumtripolyphosphat (SKTP), Kaliumdiphosphat (TKPP), saures Natriummetapolyphosphat, saures Natriumpolyphosphat, Ethylendiamintetraacetat, Aminotrimethylenphosphat oder dessen Salz, Hydroxyethylendiphosphonat oder dessen Salz, Ethylendiamintetramethylenphosphonat oder dessen Salz, Diethylentriaminpentamethylenphosphonat oder dessen Salz oder eine Mischung davon ist.

13. Zahnbleichmittel nach Anspruch 12, wobei das Peroxidstabilisierungsmittel in einem Anteil von 0,1 bis 10 Gew.-% bezogen auf das Gewicht des Peroxids vorliegt.

## Revendications

1. Blanchisseur de dents dans une phase à émulsion E/H qui comprend un monooléate de glycérol, un polyol, un polymère, un peroxyde et un solvant hydrophile, dans lequel le monooléate de glycérol est en une quantité de 15 à 95 % en poids de la composition, le polyol est en une quantité de 0,05 à 20 % en poids de la composition, et le polyol est le polyéthylèneglycol, le polypropylèneglycol, le propylèneglycol, la glycérine, l'alcool stéarylique, le xylitol, le sorbitol, le mannitol ou un mélange de ceux-ci.

2. Blanchisseur de dents selon la revendication 1, dans lequel le polyol a un poids moléculaire de 8000 ou moins.

3. Blanchisseur de dents selon la revendication 1, dans lequel le polymère est l'alcool polyvinylique, le poloxamère, la polyvinylpyrrolidone, un copolymère de polyvinylpyrrolidone/acétate de vinyle, l'hydroxypropylcellulose, l'hydroxypropylméthylcellulose, l'hydroxyéthylcellulose, l'hydroxypropyléthylcellulose, le polyox, la carboxyméthylcellulose, un polymère constitué de carboxypropylcellulose ou de son sel, la gomme de xanthane, la gomme de carraghénane, la gomme d'alginate, la gomme karaya, la gomme arabique ou son dérivé de sel, la gélatine, l'acide polyacrylique, le carbopol, le polyquaternium-11, le polyquaternium-39, un copolymère d'éther polyalkylvinylique-acide maléique (PVM/MA) ou un mélange de ceux-ci.

4. Blanchisseur de dents selon la revendication 1, dans lequel le peroxyde est le peroxyde d'hydrogène, le percarbonate, le perborate, le peroxyde d'urée, le pyrophosphate perphosphate de tétrasodium, un complexe de polyvinylpyrrolidoneperoxyde d'hydrogène, le peroxyde de calcium (CaO₂), le peroxyde de baryum (BaO₂), le peroxyde de magnésium (MgO₂), le peroxyde de sodium (Na₂O₂), le peroxyde de potassium (Ka₂O₂) ou un mélange de ceux-ci.

5. Blanchisseur de dents selon la revendication 1, dans lequel le monooléate de glycérol est en une quantité de 30 à 85 % en poids de la composition.

6. Blanchisseur de dents selon la revendication 1, dans lequel le polyol est en une quantité de 1 à 10 % en poids de la composition.

7. Blanchisseur de dents selon la revendication 1, dans lequel le polymère est en une quantité de 0,2 à 40 % en poids de la composition.

8. Blanchisseur de dents selon la revendication 7, dans lequel le polymère est en une quantité de 0,2 à 10 % en poids de la composition.

9. Blanchisseur de dents selon la revendication 1, dans lequel le solvant hydrophile est l'eau ou l'éthanol, et est en une quantité de 1 à 50 % en poids.

10. Blanchisseur de dents selon la revendication 9, dans lequel le solvant hydrophile est en une quantité de 2 à 20 % en poids de la composition.

11. Blanchisseur de dents selon la revendication 1, qui comprend en outre un stabilisant de peroxyde.

12. Blanchisseur de dents selon la revendication 11, dans lequel le stabilisant de peroxyde a une fonction de réglage de pH, et est le pyrophosphate de tétrasodium (TSPP), le dihydrogenpyrophosphate de sodium (SAPP), l'hexamétaphosphate de sodium (SHMP), le tripolyphosphate de sodium (STP), le tripolyphosphate de sodium et de potassium (SKTP), le pyrophosphate de tétrapotassium (TKPP), le métapolyphosphate sodique acide, le polyphosphate sodique acide, l'éthylènediaminetétraacétate, l'aminotriméthylènephosphate ou son sel, l'hydroxyéthylènediphosphonate ou son sel, l'éthylènediaminetétraméthylènephosphonate ou son sel, le diéthylènetriaminepentaméthylènephosphonate ou son sel, ou un mélange de ceux-ci.

13. Blanchisseur de dents selon la revendication 12, dans lequel le stabilisant de peroxyde est en une quantité de 0,1 à 10 % en poids basée sur le poids du peroxyde.
